# FASCICULE DE BREVET EUROPEEN

(11) **EP 3 531 965 B1**
(45) Date de publication et mention de la délivrance du brevet: **17.02.2021**
(21) Numéro de dépôt: 17794015.2
(22) Date de dépôt: 17.10.2017
(51) Int. Cl.: A61F 2/00, B65D 6/00, B65D 77/04

(54) **EMBALLAGE MEDICAL**
MEDIZINISCHE VERPACKUNG
MEDICAL PACKAGE

(30) Priorité: 25.10.2016 FR 1660342
(43) Date de publication de la demande: 04.09.2019
(73) Titulaire: Selenium Medical, 17000 La Rochelle (FR)
(72) Inventeur: RICHART, Olivier, 17140 Lagord (FR)
(74) Mandataire: Ipsilon
(86) Numéro de dépôt international: PCT/FR2017/052853
(87) Numéro de publication internationale: WO 2018/078242

(56) Documents cités:
- WO-A1-2014/188142
- FR-A1- 2 656 792
- US-A1- 2010 140 124

## Description

### DOMAINE DE L'INVENTION

La présente invention concerne de manière générale les emballages pour objets, en particulier pour pièces médicales de préférence stérilisées.

### ART ANTERIEUR

On connaît du document FR3005937 (ou WO2014/188142A1) un emballage et un ensemble de double emballages dont l'encombrement est réduit et dont le scellage de chaque emballage est obtenu par application d'une bande de scellage.

La présente invention a pour but de proposer un emballage et un ensemble de double emballages pour lequel ou chacun desquels la bande de scellage est applicable de manière fiable sur la zone d'ouverture dudit emballage.

### RESUME DE L'INVENTION

A cet effet, l'invention a pour objet un emballage pour un objet, de préférence médical, ledit emballage comprenant :
- une coque inférieure et
- une coque supérieure
caractérisé en ce que la coque supérieure comprend :
∘ une partie de fermeture, appelée capot,
∘ et une partie de liaison, se présentant sous la forme d'un corps traversant, et reliée au capot par une charnière ;
   la partie de liaison étant munie d'un élément de rigidification écarté de la charnière,
et en ce que ledit emballage comprend aussi
- une bande de scellage qui relie les bords du capot et de la partie de liaison qui se font face sur toute la longueur desdits bords,
- ladite partie de liaison de la coque supérieure et la coque inférieure étant deux pièces fixées l'une à l'autre, de préférence par soudage.

Une telle conception de l'emballage permet à la bande de scellage d'être appliquée sur la zone d'ouverture définie entre les bords (ou pourtours d'extrémité) du capot et de la partie de liaison qui se font face, sans risque de déformation de la zone d'ouverture définie par lesdits bords, grâce à la rigidité conférée à la coque supérieure par l'élément de rigidification.

On obtient ainsi un emballage et, comme détaillé ci-après, un ensemble de double-emballages, pour lequel ou chacun desquels la bande de scellage peut être appliquée de manière fiable au niveau de la zone d'ouverture dudit emballage, avec un risque réduit de déformer la coque, ce qui permet d'appliquer correctement la bande et donc de bénéficier d'une bonne étanchéité.

Ainsi contrairement à la solution de l'état de la technique, il n'est pas besoin de concevoir une zone d'ouverture de forme spécifique adaptée à l'utilisation d'une pièce de contre-appui pour obtenir une bonne rigidité de la coque supérieure et pouvoir appliquer correctement la bande de scellage.

Le fait de réaliser séparément la coque inférieure et la coque supérieure, en particulier la partie de liaison de la coque supérieure par rapport à la coque inférieure, permet de concevoir un même modèle de coque supérieure pour différentes formes et/ou longueurs de coque inférieure selon le type d'objet destiné à être contenu dans l'emballage.

L'opérateur peut ainsi avoir une seule référence de coque supérieure et plusieurs références de coques inférieures correspondant à une grande variété de formes et/ou de longueurs d'objet à loger dans l'emballage. Après avoir sélectionné la coque inférieure de forme adaptée à l'objet, ledit objet est introduit dans la coque inférieure puis le bord (ou pourtour) d'extrémité supérieur est soudé à l'élément de rigidification de la coque supérieure. La coque supérieure est en position fermée au sens où le capot est en regard de la partie de liaison.

Selon une caractéristique avantageuse de l'invention, le capot, la charnière et la partie de liaison de la coque supérieure sont formés d'une seule pièce par moulage.

Selon une caractéristique avantageuse de l'invention, l'élément de rigidification est aussi écarté du pourtour de l'embouchure de la coque supérieure pour permettre à la portion d'extrémité de la coque supérieure qui s'étend entre ledit élément de rigidification et le pourtour de l'embouchure, d'être introduite dans la coque inférieure.

Selon une caractéristique avantageuse de l'invention, la bande de scellage est appliquée par une de ses extrémités sur la charnière, s'étend le long des bords du capot et de la partie de liaison et revient s'appliquer sur la charnière.

Selon une caractéristique avantageuse de l'invention, la partie de liaison de la coque supérieure et la partie de la coque inférieure destinée à être soudée à ladite partie de liaison présentent une section de forme oblongue ou rectangulaire dont les coins sont arrondis.

Préférentiellement, ledit élément de rigidification est une collerette externe.

L'invention concerne également un ensemble comprenant :
- un emballage tel que décrit ci-dessus, appelé emballage intérieur, dans lequel est logé un objet, tel qu'un implant médical,
- un emballage tel que décrit ci-dessus, appelé emballage extérieur, dans lequel est logé l'emballage intérieur.

Selon une caractéristique avantageuse de l'invention, ledit ensemble comprend une coiffe de protection permettant de coiffer la coque supérieure de l'emballage extérieur, en venant, par le pourtour de son embouchure, en appui dudit élément de rigidification de la partie de liaison de la coque supérieure.

L'invention concerne également un procédé selon la revendication 9.

Selon un aspect particulier, le soudage de la coque supérieure de l'emballage extérieur est réalisé au niveau dudit élément de rigidification, de préférence une collerette, avec le pourtour d'extrémité libre de la coque inférieure de l'emballage extérieur.

Selon un aspect particulier, le soudage de la coque supérieure de l'emballage intérieur est réalisé au niveau dudit élément de rigidification, de préférence une collerette, avec le pourtour d'extrémité libre de la coque inférieure de l'emballage intérieur.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :
- la figure 1 est une vue en perspective d'un ensemble conformément à un mode de réalisation de l'invention, à l'état assemblé de l'emballage intérieur et avant assemblage de la coque supérieure et de la coque inférieure de l'emballage extérieur qui loge l'emballage intérieur ;
- la figure 2 est une vue en perspective de l'emballage intérieur qui loge un implant médical, avant assemblage de la coque supérieure et de la coque inférieure dudit emballage intérieur, conformément à un mode de réalisation de l'invention ;
- la figure 3 est une vue en perspective de la coque supérieure de l'emballage extérieur, avant application de la bande de scellage correspondante, conformément à un mode de réalisation de l'invention ;
- la figure 4 est une vue en perspective de la coque supérieure de l'emballage extérieur, après application de la bande de scellage correspondante, conformément à un mode de réalisation de l'invention ;
- la figure 5 est une vue en perspective d'un ensemble conformément à un mode de réalisation de l'invention, ledit ensemble comprenant une coiffe de protection permettant de coiffer la coque supérieure de l'emballage extérieur ;
- la figure 6 est une vue en perspective d'un autre modèle de coque inférieure de l'emballage intérieur dont la forme permet de loger un autre objet, tel qu'une prothèse de hanche, conformément à un mode de réalisation de l'invention.

### DESCRIPTION DETAILLEE

Le concept de l'invention est décrit plus complètement ci-après avec référence aux dessins joints, sur lesquels des modes de réalisation du concept de l'invention sont montrés. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. L'étendue de l'invention est par conséquent définie par les revendications jointes. Les modes de réalisation qui suivent sont examinés, par souci de simplification, en relation avec la terminologie et la structure d'un emballage et d'un double emballage.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

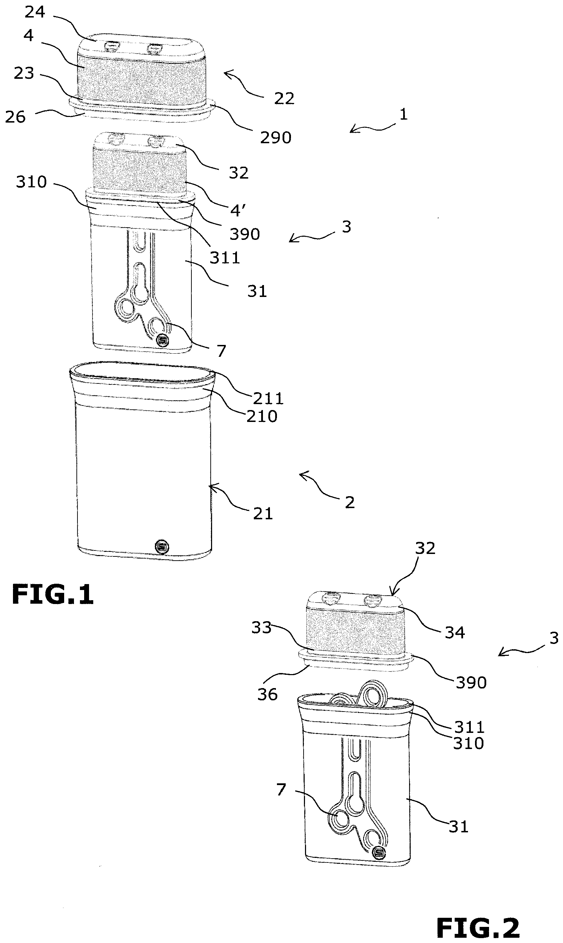

En référence aux figures, on a représenté un ensemble 1 qui comprend un premier emballage 3, dit emballage intérieur, contenant un implant médical 7 et un deuxième emballage 2, dit emballage extérieur, dans lequel est logé le premier emballage. En variante, on peut prévoir que l'emballage 3 intérieur contienne un objet autre qu'un implant médical 7. L'emballage 3 intérieur peut contenir d'autre(s) pièce(s) médicale(s) de préférence stérilisée(s) que celle illustrée aux figures 1 et 2.

En particulier, l'ensemble est conçu à des fins de préservation du caractère stérile dudit objet et en vue du déballage de l'objet dans des conditions aseptiques ou proches de l'asepsie. Ledit objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés par exemple par rayonnement.

L'emballage intérieur et l'emballage extérieur comprennent des caractéristiques identiques ou similaires, à l'exception bien entendu de leurs dimensions.

Ainsi, la description qui suit est appliquée à l'emballage 2 extérieur dans lequel est logé ledit emballage 3 intérieur, mais s'applique aussi à l'emballage 3 intérieur qui contient l'implant médical.

Dans l'exemple illustré aux figures, l'emballage 2 extérieur comprend une coque inférieure 21 et une coque supérieure 22. La coque inférieure 21 se présente sous la forme d'un corps creux présentant une extrémité fermée formant fond et une extrémité opposée qui est ouverte. Ainsi chaque coque est considérée comme une coque ouverture au sens où elle forme une cavité borgne et permet à l'état assemblé avec l'autre de définir une chambre fermée à l'état appliqué de la bande de scellage comme détaillé ci-après. La portion d'extrémité ouverte 210 est évasée de manière à recevoir une partie de la coque supérieure.

La coque supérieure 22 comprend une partie de fermeture 24, appelée capot, et une partie, appelée partie de liaison 23, qui se présente sous la forme d'un corps traversant. En particulier ladite partie de liaison se présente sous la forme d'un tronçon de la coque supérieure. La partie de liaison est de forme générale cylindrique dont la paroi périphérique se développe autour de l'axe d'ouverture de ladite coque supérieure.

La partie de liaison 23 est reliée au capot 24 par une charnière 25 pour permettre d'articuler le capot 24 par rapport à la partie de liaison 23 entre une position relevée (ouverte) libérant l'accès à l'intérieur de la partie de liaison et une postions abaissée (fermée) empêchant l'accès à l'intérieur de ladite partie de liaison.

Une fois la coque supérieure soudée à la coque inférieure, la charnière entre le capot et la liaison de la coque supérieure permet d'ouvrir le capot pour libérer l'accès à l'intérieur de la coque inférieure 21 à travers la partie de liaison 23.

La charnière relie une portion du pourtour supérieur de la partie de liaison au pourtour du capot. Le pourtour inférieur de la partie de liaison est emboité dans la portion d'extrémité évasée de la coque inférieure 21.

Le pourtour du capot est de même forme et de même dimension que le pourtour supérieur du corps de liaison. Ainsi en position fermée du capot, lesdits pourtours sont situés en regard l'un de l'autre et forme ensemble ladite coque supérieure en présentant une extrémité fermée et une extrémité opposée ouverte qui communique avec la coque inférieure.

La paroi périphérique de la partie de liaison 23 est munie d'une collerette 290, de préférence située à proximité du pourtour inférieur de la coque supérieure qui délimite l'ouverture de la coque supérieure. Dans l'exemple illustré aux figures, ladite collerette est une collerette externe.

La collerette 290 est écartée de la zone d'ouverture de la coque supérieure délimitée entre le pourtour supérieur 231 de la partie de liaison et le pourtour 241 correspondant du capot pour permettre à la bande de scellage 4 de recouvrir la zone d'ouverture définie entre lesdits pourtours.

La collerette 290 est destinée à venir en applique contre le pourtour d'extrémité 211 de l'ouverture de la coque inférieure. La collerette forme ainsi non seulement un élément de rigidification de la coque supérieure mais aussi une butée de limitation d'emboîtement de la coque supérieure dans la coque inférieure. La coque inférieure et la coque supérieure sont destinées être soudées, de préférence au niveau de la collerette de la coque supérieure qui est appliquée sur le pourtour d'extrémité libre de la coque inférieure. La face de la collerette opposée à celle destinée à venir en applique du pourtour d'extrémité libre de la coque inférieure permet de recevoir le pourtour d'extrémité de l'ouverture de la coiffe 9 comme expliqué ci-après.

Dans l'exemple illustré aux figures, la collerette 290 est écartée du pourtour inférieur de la coque supérieure qui délimite l'embouchure de la coque supérieure, pour laisser une portion de la paroi périphérique, appelée portion d'engagement 26, de la coque supérieure, être insérée dans la coque inférieure, en particulier dans la portion évasée de la coque inférieur comme présenté ci-après. La portion d'engagement de la coque supérieure et la portion d'extrémité libre de la coque inférieure sont configurées pour permettre un emboitement de ladite portion d'engagement de la coque supérieure dans ladite portion d'extrémité libre de la coque inférieure, la collerette formant butée d'arrêt lors de cet emboitement.

Avantageusement, la section de passage définie par la portion d'engagement (qui forme l'ouverture de la coque supérieure) est plus grande que la section de passage délimitée par la partie de liaison mais inférieure à la surface délimitée par le pourtour extérieur de la collerette

Comme évoqué ci-dessus, la bande de scellage 4 relie, du côté extérieur de la coque supérieure, les bords du capot et de la partie de liaison qui se font face à l'état fermé (ou abaissé) du capot, sur toute la longueur des bords des coques de l'emballage et d'un bord à l'autre de manière à sceller l'emballage.

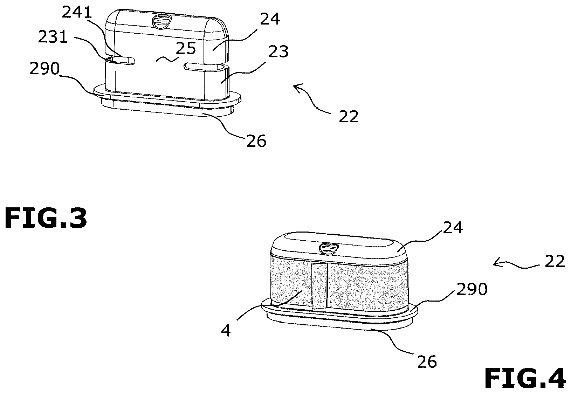

Préférentiellement, la bande de scellage 4 est appliquée par une de ses extrémités sur la charnière 25, s'étend le long des bords 241, 231 respectivement du capot 24 et de la partie de liaison 23, qui s'étendent en regard l'une de l'autre, et revient s'appliquer sur la charnière 25. Comme illustré à la figure 3, chaque bord s'arrête 241, 231 à la charnière 25 de liaison des coques entre elles.

On obtient ainsi une très bonne étanchéité aux bactéries et au liquide, sans discontinuité le long de la zone d'ouverture de la coque supérieure. A l'inverse, un chevauchement d'une extrémité de la bande par elle-même au niveau d'une partie de la zone d'ouverture de l'emballage, risquerait de provoquer une mauvaise étanchéité.

Préférentiellement, la bande de scellage 4 est une bande pelable. Une fois la bande appliquée, celle-ci peut être retirée en tirant dessus pour la peler. On entend par pelable le fait que la bande ne se déchire pas lorsqu'on la retire, tandis que la colle reste sur la zone des coques précédemment scellée.

Selon un aspect particulier, la bande de scellage est appliquée sur l'emballage à chaud et sous pression.

Selon un mode de réalisation ladite bande est thermocollante par exemple en matériau synthétique non-tissé fabriquée à partir de fibres de polyéthylène, usuellement vendue sous la marque déposée TYVEK. On peut prévoir de remplacer cette solution de scellage à chaud sous pression par une autre solution de scellage, par exemple à l'aide d'une colle chimique adaptée.

Dans le cas d'une bande thermocollante, la bande de scellage 4 est appliquée sur chaque emballage 2, 3 à chaud et sous pression. La bande de scellage 4 comprend une face munie de colle qui devient active lorsqu'elle est appliquée sur le support à sceller avec une pression et une température donnée.

En variante, on peut prévoir que la bande soit déchirable.

Une fois assemblées, ladite partie de liaison 23 de la coque supérieure 22 et la coque inférieure 21 forment deux pièces fixées l'une à l'autre de manière indémontable par l'utilisateur. Préférentiellement, la fixation est réalisée par soudage entre la collerette et le pourtour d'extrémité libre de la coque inférieure.

Autrement dit, la partie de liaison 23 de la coque supérieure et la coque inférieure 21 ne sont pas formées d'une seule pièce, mais sont réalisées séparément, puis fixées l'une à l'autre de manière indémontable, c'est-à-dire de sorte qu'elles ne soient pas séparables l'une de l'autre par l'utilisateur.

Le capot 24 et la partie de liaison 23 de la coque supérieure 22 sont formés d'une seule pièce par moulage. La forme du moule est choisie de sorte que le capot soit en configuration fermée (c'est-à-dire abaissée) du capot, ce qui est rendu possible par la réalisation séparée de la coque inférieure, de sorte que la coque supérieure puisse être démoulée grâce à son ouverture délimitée par le pourtour d'extrémité libre qui définit l'embouchure de la coque supérieure, tout en présentant une charnière 25 entre le capot 24 et la partie de liaison 23. La partie de liaison 23 et la coque inférieure 21 sont par la suite fixées entre elles de manière inséparable comme rappelé ci-dessus.

Dans l'exemple illustré aux figures, le capot 24, la partie de liaison 23 et la charnière 25 qui les relie sont formés d'une seule pièce, de préférence en plastique.

La partie de liaison 23 de la coque supérieure 22 et le bord d'extrémité libre 211 de la partie 210 de la coque inférieure 21 destinée à être soudée à ladite partie de liaison 23 présentent une section de forme oblongue ou rectangulaire dont les coins sont arrondis pour faciliter le démoulage.

Comme expliqué ci-avant, la description réalisée ci-dessous pour l'emballage 2 s'applique aussi à l'emballage 3 qui est logé dans l'emballage 2 et qui contient lui-même l'implant médical 7. Ainsi, la description ci-dessus peut être lue en remplaçant dans les références citées, le chiffre « 2 » par le chiffre « 3 » pour obtenir les références correspondant à l'emballage 3. A noter que la bande de scellage 4 décrite dans le cadre de l'emballage 2 correspond pour l'emballage 3 à la bande de scellage 4'.

Ainsi, l'emballage 3 intérieur comprend une coque inférieure 31 présentant une portion d'extrémité ouverte 310 et un pourtour d'extrémité libre 311, et une coque supérieure 32 qui comprend un capot 34, une partie de liaison 33 munie d'une collerette 390, une charnière (masquée par la bande de scellage 4' correspondante dans les figures 1 et 2), et une portion d'engagement 36.

Selon un mode de réalisation non illustré aux figures, ledit ensemble comprend un premier emballage, ou emballage intérieur, supplémentaire logé dans l'emballage extérieur à côté de l'autre emballage intérieur. Avantageusement, les emballages présentent une section de forme générale rectangulaire ou carrée.

Ledit emballage 2 extérieur comprend une coiffe de protection 9 permettant de coiffer la coque supérieure 21 de l'emballage 2 extérieur, en venant, par le pourtour 900 de son embouchure, en appui de la collerette externe 290 de la partie de liaison 23 de la coque supérieure 22.

Chaque emballage présenté ci-dessus peut être réalisé selon un procédé de fabrication qui comprend les étapes suivantes. La coque supérieure 22 (respectivement 32) est moulée d'une seule pièce et séparément de la coque inférieure 21 (respectivement 31). La forme du moule correspond à une configuration de la coque supérieure selon laquelle le capot est en position dite fermée c'est-à-dire en regard de la partie de liaison.

L'embouchure de la coque supérieure définie par le pourtour d'extrémité libre de la partie de liaison situé du côté de la partie de liaison opposé à la charnière, permet de démouler aisément la coque supérieure, ce qui ne serait pas possible si la coque inférieure et la coque supérieure étaient réalisées d'une seule pièce.

Ainsi la coque supérieure peut être aisément obtenue par moulage et dans une configuration selon laquelle le capot est en position abaissée en regard de la partie de liaison. Tant que la coque inférieure et la coque supérieure de l'un ou l'autre des emballages ne sont pas soudées entre elles, il est possible d'introduire l'objet souhaité dans l'emballage correspondant sans avoir à manipuler la charnière.

De même, après soudage de l'emballage intérieur, il est possible d'introduire l'emballage intérieur soudé dans l'emballage extérieur non encore soudé, là encore sans avoir à solliciter la charnière. Puis les coques inférieure et supérieures de l'emballage extérieur sont soudées. Pour chaque emballage le soudage correspond au soudage de la coque supérieure au niveau de sa collerette externe avec le pourtour d'extrémité libre de la coque inférieure. Le fait de ne pas avoir à solliciter la charnière lors de la réalisation de chaque emballage et de l'ensemble correspondant permet de réduire le risque de dégradation de chaque charnière qui est formée en plastique d'une seule pièce avec le reste de la coque supérieure de l'emballage correspondant.

Pour déballer l'objet un opérateur doit défaire la bande de scellage, ouvrir l'emballage extérieur en articulant le capot à l'aide la charnière plastique, puis extraire l'emballage intérieur et répéter ces opérations pour accéder à l'objet contenu dans l'emballage intérieur.

Le fait que la charnière soit en plastique, permet de destiner l'emballage correspondant à un usage unique du fait que la sollicitation de la charnière plastique lors de l'ouverture du capot, la déforme de sorte qu'elle ne reprend pas sa position de fermeture d'origine. Autrement dit, l'ouverture du capot entraine la déformation plastique de la charnière de sorte que, une fois ouvert, le capot ne peut pas être refermé correctement, ce qui permet d'obtenir un emballage à usage unique. En variante, la charnière peut être sécable.

Selon un mode de réalisation, les coques inférieures et/ou les coques supérieures sont réalisées en un matériau translucide, de préférence transparent.

Selon un mode de réalisation, la zone d'ouverture de l'un ou de chaque emballage, définie entre les bords des coques dudit emballage, est plus proche du fond d'une coque que du fond de l'autre coque dudit emballage.

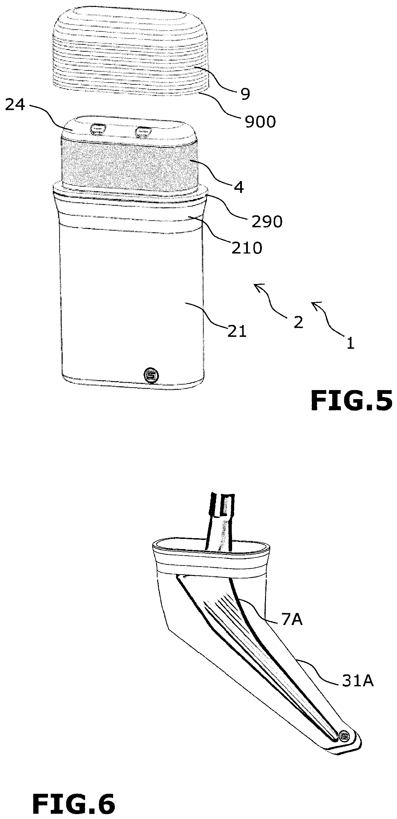

Comme expliqué ci-avant et comme illustré à la figure 6, il est possible de fixer à la coque supérieure un modèle de coque inférieure autre que celui présenté aux figures 1 et 2. Ainsi, dans l'exemple illustré à la figure 6, la coque inférieure 31A de l'emballage intérieur présente une forme générale en L adaptée à loger une prothèse de hanche 7A. Dans ce cas, la coque inférieure de l'emballage extérieur (non représentée) est elle aussi en forme générale de L pour pouvoir loger la coque inférieure de l'emballage intérieur.

L'invention n'est pas limitée aux modes de réalisation illustrés dans les dessins. En conséquence, il doit être entendu que, lorsque les caractéristiques mentionnées dans les revendications annexées sont suivies par des signes de référence, ces signes sont inclus uniquement dans le but d'améliorer l'intelligibilité des revendications et ne sont nullement limitatifs de la portée des revendications.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Emballage (2; 3) pour un objet médical, ledit emballage comprenant
- une coque inférieure (21 ; 31) et
- une coque supérieure (22 ;32)
**caractérisé en ce que** la coque supérieure (22 ;32) comprend :
∘ une partie de fermeture (24 ;34), appelée capot,
∘ et une partie de liaison (23 ;33), se présentant sous la forme d'un corps traversant, et reliée au capot (24 ; 34) par une charnière (25) ;
la partie de liaison (23 ; 33) étant munie d'un élément de rigidification (290 ; 390) écarté de la charnière,
et **en ce que** ledit emballage comprend aussi
- une bande de scellage (4 ; 4') qui relie les bords (241, 231) du capot (24 ; 34) et de la partie de liaison (23 ; 33) qui se font face sur toute la longueur desdits bords et d'un bord à l'autre de manière à sceller l'emballage,
- ladite partie de liaison (23 ; 33) de la coque supérieure (22 ; 32) et la coque inférieure (21 ; 31) étant deux pièces fixées l'une à l'autre, de préférence par soudage.

2. Emballage (2 ; 3) selon la revendication 1, **caractérisé en ce que** le capot (24 ; 34), la charnière (25) et la partie de liaison (23 ; 33) de la coque supérieure (22 ; 32) sont formés d'une seule pièce par moulage.

3. Emballage (2 ; 3) selon l'une des revendications précédentes, **caractérisé en ce que** ledit élément de rigidification (290 ; 390) est aussi écarté du pourtour de l'embouchure de la coque supérieure (22 ; 32) pour permettre à la portion d'extrémité (26 ; 36) de la coque supérieure qui s'étend entre ledit élément de rigidification (290 ; 390) et le pourtour de l'embouchure, d'être introduite dans la coque inférieure (21 ; 31).

4. Emballage (2 ; 3) selon l'une des revendications précédentes, **caractérisé en ce que** la bande de scellage (4) est appliquée par une de ses extrémités sur la charnière (25), s'étend le long des bords (241, 231) du capot (24) et de la partie de liaison (23) et revient s'appliquer sur la charnière (25).

5. Emballage (2 ; 3) selon l'une des revendications précédentes, **caractérisé en ce que** la partie de liaison (23 ; 33) de la coque supérieure (22 ; 32) et la partie de la coque inférieure (21 ; 31) destinée à être soudée à ladite partie de liaison (23 ; 33) présentent une section de forme oblongue ou rectangulaire dont les coins sont arrondis.

6. Emballage (2 ; 3) selon l'une des revendications précédentes, **caractérisé en ce que** ledit élément de rigidification (290, 390) est une collerette externe.

7. Ensemble (1) comprenant :
- un emballage (3) conforme à l'une des revendications précédentes, appelé emballage intérieur, dans lequel est logé un objet (7), tel qu'un implant médical,
- un emballage (2) conforme à l'une des revendications précédentes, appelé emballage extérieur, dans lequel est logé l'emballage (3) intérieur.

8. Ensemble (1) selon la revendication 7, **caractérisé en ce que** ledit ensemble comprend une coiffe de protection (9) permettant de coiffer la coque supérieure (22) de l'emballage (2) extérieur, en venant, par le pourtour (900) de son embouchure, en appui dudit élément de rigidification (290) de la partie de liaison (23) de la coque supérieure (22).

9. Procédé de fabrication d'un ensemble (1) conforme à l'une des revendications 7 et 8, **caractérisé en ce que** ledit procédé comprend les étapes suivantes :
- fabrication d'un emballage intérieur (3) et d'un emballage extérieur (2), comprenant pour chacun des emballages intérieur (3) et extérieur (2), les étapes suivantes de :
- moulage d'une coque supérieure (22 ; 32) d'une seule pièce et séparément d'une coque inférieure (21 ; 31),
la coque supérieure (22 ;32) comprenant une partie de fermeture (24 ;34), appelée capot,
et une partie de liaison (23 ;33), se présentant sous la forme d'un corps traversant, et reliée au capot (24 ; 34) par une charnière (25) ;
la partie de liaison (23 ; 33) étant munie d'un élément de rigidification (290 ; 390) écarté de la charnière,
le capot (24 ; 34) de la coque supérieure se présentant en position fermée ; et
- démoulage de la coque supérieure (22 ; 32), le capot (24 ; 34) étant en position fermée ;
ledit procédé comprenant en outre les étapes suivantes :
- introduction d'un objet (7) dans la coque inférieure (31) de l'emballage intérieur (3) ;
- soudage de la coque supérieure (32) de l'emballage intérieur (3) avec la coque inférieure (31) de l'emballage intérieur (3);
- avant ou après l'étape de soudage, application d'une bande de scellage (4') sur la coque supérieure (32) de l'emballage intérieur (3) ;
- introduction de l'emballage intérieur (3) dans la coque inférieure (21) de l'emballage extérieur (2) ;
- soudage de la coque supérieure (22) de l'emballage extérieur (2) avec la coque inférieure (21) de l'emballage extérieur (2) ;
- avant ou après l'étape de soudage, application d'une bande de scellage (4) sur la coque supérieure (22) de l'emballage extérieur (2).

## Patentansprüche

1. Verpackung (2; 3) für einen medizinischen Gegenstand, wobei die Verpackung umfasst
- eine untere Schale (21; 31) und
- eine obere Schale (22; 32)
**dadurch gekennzeichnet, dass** die obere Schale (22; 32) umfasst:
∘ einen Verschlussteil (24; 34), bezeichnet als Deckel,
∘ und einen Verbindungsteil (23; 33), der in Form eines Durchgangskörpers vorliegt und mit dem Deckel (24; 34) durch ein Scharnier (25) verbunden ist;
wobei der Verbindungsteil (23; 33) mit einem von dem Scharnier beabstandeten Versteifungselement (290; 390) versehen ist,
und dass die Verpackung ebenfalls umfasst
- ein Versiegelungsband (4; 4'), das die Ränder (241, 231) des Deckels (24; 34) und des Verbindungsteils (23; 33) verbindet, die sich über die gesamte Länge der Ränder gegenüberliegen und von einem Rand zum anderen, so dass die Verpackung versiegelt wird,
- wobei der Verbindungsteil (23; 33) der oberen Schale (22; 32) und die untere Schale (21; 31) zwei Teile sind, die aneinander vorzugsweise durch Schweißen befestigt sind.

2. Verpackung (2; 3) nach Anspruch 1, **dadurch gekennzeichnet, dass** der Deckel (24; 34), das Scharnier (25) und der Verbindungsteil (23; 33) der oberen Schale (22; 32) aus einem einzigen Teil durch Formen gebildet sind.

3. Verpackung (2; 3) nach einem der vorangehenden Ansprüche, **dadurch gekennzeichnet, dass** das Versteifungselement (290; 390) auch vom Umfang der Mündung der oberen Schale (22; 32) beabstandet ist, um dem Endabschnitt (26; 36) der oberen Schale, der sich zwischen dem Versteifungselement (290; 390) und dem Umfang der Mündung erstreckt, zu gestatten, in die untere Schale (21; 31) eingeführt zu sein.

4. Verpackung (2; 3) nach einem der vorangehenden Ansprüche, **dadurch gekennzeichnet, dass** das Versiegelungsband (4) mit einem seiner Enden auf dem Scharnier (25) anliegt, sich entlang der Ränder (241, 231) des Deckels (24) und des Verbindungsteils (23) erstreckt und zurückkehrt, um auf dem Scharnier (25) anzuliegen.

5. Verpackung (2; 3) nach einem der vorangehenden Ansprüche, **dadurch gekennzeichnet, dass** der Verbindungsteil (23; 33) der oberen Schale (22; 32) und der Teil der unteren Schale (21; 31), der bestimmt ist, mit dem Verbindungsteil (23; 33) verschweißt zu sein, einen länglichen oder viereckigen Querschnitt aufweisen, dessen Ecken abgerundet sind.

6. Verpackung (2; 3) nach einem der vorangehenden Ansprüche, **dadurch gekennzeichnet, dass** das Versteifungselement (290, 390) ein äußerer Kragen ist.

7. Einheit (1), umfassend:
- eine Verpackung (3) nach einem der vorangehenden Ansprüche, bezeichnet als innere Verpackung, in der ein Gegenstand (7) wie ein medizinisches Implantat untergebracht ist,
- eine Verpackung (2) nach einem der vorangehenden Ansprüche, bezeichnet als äußere Verpackung, in der die innere Verpackung (3) untergebracht ist.

8. Einheit (1) nach Anspruch 7, **dadurch gekennzeichnet, dass** die Einheit eine Schutzkappe (9) umfasst, die erlaubt, die obere Schale (22) der äußeren Verpackung (2) abzudecken, indem sie sich über den Umfang (900) ihrer Mündung auf dem Versteifungselement (290) des Verbindungsteils (23) der oberen Schale (22) abstützt.

9. Verfahren zur Herstellung einer Einheit (1) nach einem der Ansprüche 7 und 8, **dadurch gekennzeichnet, dass** das Verfahren die folgenden Schritte umfasst:
- Herstellen einer inneren Verpackung (3) und einer äußeren Verpackung (2), umfassend jeweils für die eine innere (3) und äußere Verpackung (2) die folgenden Schritte:
- Formen einer oberen Schale (22; 32) aus einem einzigen Teil und separat von einer unteren Schale (21; 31),
wobei die obere Schale (22, 32) einen Verschlussteil (24; 34) umfasst, bezeichnet als Deckel,
und einen Verbindungsteil (23; 33), der in Form eines Durchgangskörpers vorliegt und mit dem Deckel (24; 34) durch ein Scharnier (25) verbunden ist;
wobei der Verbindungsteil (23, 33) mit einem vom Scharnier beabstandeten Versteifungselement (290, 390) versehen ist,
wobei der Deckel (24; 34) der oberen Schale in geschlossener Position vorliegt; und
- Entformen der oberen Schale (22; 32), wobei der Deckel (24; 34) in geschlossener Position ist;
wobei das Verfahren ferner die folgenden Schritte umfasst:
- Einsetzen eines Gegenstands (7) in die untere Schale (31) der inneren Verpackung (3),
- Verschweißen der oberen Schale (32) der inneren Verpackung (3) mit der unteren Schale (31) der inneren Verpackung (3);
- vor oder nach dem Schweißschritt, Anlegen eines Versiegelungsbands (4') auf die obere Schale (32) der inneren Verpackung (3);
- Einsetzen der inneren Verpackung (3) in die untere Schale (21) der äußeren Verpackung (2),
- Verschweißen der oberen Schale (22) der äußeren Verpackung (2) mit der unteren Schale (21) der äußeren Verpackung (2);
- vor oder nach dem Schweißschritt, Anlegen eines Versiegelungsbands (4) auf die obere Schale (22) der äußeren Verpackung (2).

## Claims

1. Packaging (2; 3) for a medical object, said packaging comprising:
- a lower shell (21; 31) and
- an upper shell (22; 32)
**characterized in that** the upper shell (22; 32) comprises:
∘ a closing part (24; 34), called cover,
∘ and a connecting part (23; 33), assuming the form of a through body, and connected to the cover (24; 34) by a hinge (25);
the connecting part (23; 33) being provided with a stiffening element (290; 390) separated from the hinge,
and **in that** said package also comprises
- a sealing strip (4; 4') which connects the edges (241, 231) of the cover (24; 34) and the connecting part (23; 33) which face one another over the entire length of said edges and from one edge to the other so as to seal the package,
- said connecting part (23; 33) of the upper shell (22; 32) and the lower shell (21; 31) being two parts attached to one another, preferably by welding.

2. The package (2; 3) according to claim 1, **characterized in that** the cover (24; 34), the hinge (25) and the connecting part (23; 33) of the upper shell (22; 32) are formed in one piece by molding.

3. The package (2; 3) according to one of the preceding claims, **characterized in that** said stiffening element (290; 390) is also separated from the perimeter of the mouth of the upper shell (22; 32) so as to allow the end portion (26; 36) of the upper shell which extends between said stiffening element (290; 390) and the perimeter of the mouth to be introduced into the lower shell (21; 31).

4. The package (2; 3) according to one of the preceding claims, **characterized in that** the sealing strip (4) is applied by one of its ends on the hinge (25), extends along the edges (241, 231) of the cover (24) and of the connecting part (23) and is applied on the hinge (25).

5. The package (2; 3) according to one of the preceding claims, **characterized in that** the connecting part (23; 33) of the upper shell (22; 32) and the part of the lower shell (21; 31) which is intended to be welded to said connecting part (23; 33) have an oblong or rectangular section whose corners are rounded.

6. The package (2; 3) according to one of the preceding claims, **characterized in that** said stiffening element (290, 390) is an outer collar.

7. An assembly (1) comprising:
- a package (3) according to one of the preceding claims, called inner package, in which an object (7) is housed, such as a medical implant,
- a package (2) according to one of the preceding claims, called outer package, in which the inner package (3) is housed.

8. The assembly (1) according to claim 7, **characterized in that** said assembly comprises a protective cap (9) making it possible to cap the upper shell (22) of the outer package (2), by bearing, by the perimeter (900) of its mouth, on said stiffening element (290) of the connecting part (23) of the upper shell (22).

9. A method for manufacturing an assembly (1) according to one of claims 7 and 8, **characterized in that** said method comprises the following steps:
- manufacturing an inner package (3) and an outer package (2), comprising, for each of the inner (3) and outer (2) packages, the following steps:
- molding an upper shell (22; 32) in a single piece and separately from a lower shell (21; 31),
the upper shell (22; 32) comprising a closing part (24; 34), called cover,
and a connecting part (23; 33), assuming the form of a through body, and connected to the cover (24; 34) by a hinge (25);
the connecting part (23; 33) being provided with a stiffening element (290; 390) separated from the hinge,
the cover (24; 34) of the upper shell assuming a closed position; and
- stripping the upper shell (22; 32), the cover (24; 34) being in the closed position;
said method further comprising the following steps:
- introducing an object (7) into the lower shell (31) of the inner package (3);
- welding the upper shell (32) of the inner package (3) with the lower shell (31) of the inner package (3);
- before or after the welding step, applying a sealing strip (4') on the upper shell (32) of the inner package (3);
- introducing the inner package (3) into the lower shell (21) of the outer package (2);
- welding the upper shell (22) of the outer package (2) with the lower shell (21) of the outer package (2);
- before or after the welding step, applying a sealing strip (4) on the upper shell (22) of the outer package (2).
